# EUROPÄISCHE PATENTANMELDUNG

(11) **EP 1 967 315 A1**
(43) Veröffentlichungstag der Anmeldung: **10.09.2008**
(21) Anmeldenummer: 07023413.3
(22) Anmeldetag: 04.12.2007
(51) Int. Cl.: B23K 26/00, A61N 1/375, B29C 65/14, B29C 65/48, C08J 5/12, C09J 5/02

(54) **Medizinisches Implantat und Verfahren zur Herstellung desselben**

(30) Priorität: 06.03.2007 DE 102007011310
(71) Anmelder: BIOTRONIK CRM Patent AG, 6341 Baar (CH)
(72) Erfinder: Litzke, Jan, 13507 Berlin (DE)
(74) Vertreter: Lindner-Vogt, Karin L.

(57) **Zusammenfassung**

Die vorliegende Erfindung bezieht sich auf ein Verfahren zur Herstellung eines medizinischen Implantats, vorzugsweise eines Herzschrittmachers oder eines Defibrillators. Ein derartiges medizinisches Implantat wird aus einem ersten Element (20,30) und mindestens einem zweiten Element (20,30), die jeweils eine Oberfläche aufweisen, zusammengesetzt. Das erste Element (20,30) und das mindestens eine zweite Element (20,30) werden dabei in einem Fügeschritt in dem jeweiligen, mindestens einen Teilbereich der jeweiligen Oberfläche bildenden Verbindungsbereich des ersten Elements (20,30) und des mindestens einen zweiten Elements (20,30) miteinander verbunden. In einem vor dem Fügeschritt durchzuführenden Plasmaaktivierungsschritt wird mindestens der Verbindungsbereich des ersten Elements (20,30) und/oder mindestens der Verbindungsbereich des mindestens einen zweiten Elementes (20,30) in einem Niederdruckplasma oder in einem bei Atmosphärendruck erzeugten Plasma (51) aktiviert. Die Erfindung betrifft außerdem ein entsprechend hergestelltes medizinisches Implantat.

## Beschreibung

Die vorliegende Erfindung betrifft ein Verfahren zur Herstellung eines medizinischen Implantats, vorzugsweise eines Herzschrittmachers oder eines Defibrillators, bestehend aus einem ersten Element und mindestens einem zweiten Element, die jeweils eine Oberfläche aufweisen, wobei das erste Element und das mindestens eine zweite Element in einem Fügeschritt in dem jeweiligen, mindestens einen Teilbereich der jeweiligen Oberfläche bildenden Verbindungsbereich des ersten Elements und des mindestens einen zweiten Elements miteinander verbunden werden. Die vorliegende Erfindung betrifft außerdem ein entsprechendes medizinisches Implantat.

Medizinische Implantate haben die Aufgabe, Körperfunktionen zu unterstützen oder zu ersetzen. Sie werden je nach ihrer Funktion auch als (implantierbare) Prothesen bezeichnet. Häufig verwendete medizinische Implantate sind beispielsweise Herzschrittmacher, Defibrillatoren, Hirnschrittmacher, Herzimplantate, Implantate zur operativen Behandlung von Knochenbrüchen oder Stents.

Derartige Implantate werden während ihrer Herstellung meist aus mehreren Elementen zusammengefügt. Als Fügeverfahren für diese Elemente zur Herstellung eines medizinischen Implantats eignen sich insbesondere die Fügeverfahren Zusammensetzen, Füllen, An- und Einpressen, Schweißen, Löten und Kleben sowie die Verfahren Verschrauben, Vernieten, Clinchen und Schrumpfen. Für derartige Fügeverfahren, insbesondere für das Kleben, ist der Zustand der Oberfläche der jeweiligen zu fügenden Elemente in dem Teilbereich, in dem die Elemente gefügt werden (im Folgenden Verbindungsbereich genannt), von Bedeutung, um eine optimale Festigkeit der Fügeverbindung und die Abdichtung gegenüber Körperflüssigkeiten zu gewährleisten.

Die Behandlung von Oberflächen im Bereich medizinischer Implantate ist aus dem Dokument US 6,101,973 bekannt. Das dort dargestellte Verfahren dient dazu, die Gleiteigenschaften von Polymer-Oberflächen zu verbessern. In diesem Dokument werden insbesondere Oberflächen von Schlauchmaterialien, wie beispielsweise Silikon, Polypropylen, Polyethylen, Polyvinylchlorid, Fluorpolymer oder anderen dielektrischen Materialien angesprochen. Das Dokument beschreibt, dass eine Verbesserung der Gleiteigenschaften der Oberfläche des Schlauchmaterials dadurch erreicht werden kann, indem das Material mit einer Glimmentladung in einem Plasma behandelt wird, wobei die Glimmentladung mit der Aufbringung eines Monomers verbunden ist. Hierbei kann das Schlauchmaterial in einer Vorbehandlungszone vor dem Aufbringen des Monomers mit einer weiteren Glimmentladung behandelt werden, um das Schlauchmaterial für die Aufbringung des Monomers in einer darauf folgenden Zone vorzubereiten. Als Monomere werden beispielsweise gesättigte zyklische Gruppen, Amingruppen, Hydroxylgruppen, Carbonylgruppen etc. verwendet. Ein besonders bevorzugtes Monomer ist N-Vinyl-2-Pyrrolidon (NVP). Das in US 6,101,973 angegebene Verfahren ist sehr aufwendig und zur Vorbereitung des Fügens von Elementen medizinischer Implantate nicht anwendbar, da durch die verbesserte Gleitfähigkeit der Oberfläche bspw. die Haftwirkung eines für das Fügen verwendeten Klebemittels verringert und hierdurch die Festigkeit der Verbindung negativ beeinflusst werden würde.

Elemente von medizinischen Implantaten, z. B. Teile eines Herzschrittmachers, werden bisher überwiegend von Hand montiert und anschließend teilautomatisch mittels Silikon oder anderen Klebemittel verklebt. Dieses Verfahren ist instabil und sehr aufwendig und erfordert eine Reihe von Nachbearbeitungsschritten, z. B. Polieren. Bei dem Klebeverfahren können z. B. durch unregelmäßigen Klebemittel-Auftrag Bereiche entstehen, durch die im Bereich der Klebeverbindung eine verringerte Abdichtung oder Kurzschlüsse an den Anschlüssen verursacht werden können.

Die Aufgabe der vorliegenden Erfindung besteht demnach darin, ein Verfahren zur Herstellung eines medizinischen Implantats anzugeben, das weniger Nachbearbeitung erfordert und hierdurch geringe Durchlaufzeiten ermöglicht und eine höhere Prozesssicherheit gewährleistet. Die Aufgabe besteht ferner darin, ein medizinisches Implantat zu schaffen, das in der Herstellung kostengünstiger ist und eine geringere Fehleranfälligkeit aufweist.

Diese Aufgabe wird erfindungsgemäß durch ein Verfahren der eingangs genannten Art zur Herstellung eines medizinischen Implantats gelöst, bei dem in einem vor dem Fügeschritt durchzuführenden Plasmaaktivierungsschritt mindestens der Verbindungsbereich des ersten Elements und/oder mindestens der Verbindungsbereich des mindestens einen zweiten Elements in einem Niederdruckplasma oder in einem bei Atmosphärendruck erzeugten Plasma (Normaldruckplasma) aktiviert wird.

Das Niederdruckplasma bzw. das bei Atmosphärendruck erzeugte Plasma ist jeweils ein ionisiertes Gas, das einen nennenswerten Anteil freier Ladungsträger (Ionen oder Elektronen) enthält. Das Niederdruckplasma wird bei einem Druck von etwa 0,1 mbar bis 1 mbar und das bei Athmosphärendruck erzeugte Plasma bei einem Druck von etwa 0,5 bar bis 8 bar erzeugt.

Das Plasma wird allgemein dadurch erzeugt, dass eine hohe elektrische Spannung in einem Gas zwischen zwei Elektroden angelegt wird. Bei einer geeigneten Kombination von Spannung, Elektrodenabstand und Gasdruck kommt es zu einem Überschlag und dem Zünden einer Entladung zwischen den Elektroden. Hierdurch werden mittels Stoßionisation ionisierte Teilchen erzeugt, die bei ihrem Weg zu den Elektroden auf ein in dem Plasma angeordnetes Element, in diesem Fall ein Element eines medizinischen Implantats, auftreffen. Hierdurch werden auf der Oberfläche des Elements polare Gruppen erzeugt oder eingebaut bzw. die Oberfläche gereinigt. Hierdurch wird die behandelte Oberfläche des Elements aktiviert, d. h. die Oberfläche weist nach der Plasmaaktivierung eine höhere Oberflächenenergie auf. Ggf. können auch die in dem Plasma enthaltenen Gasteilchen an der Oberfläche des Elements angereichert werden. Die Oberfläche wird hierdurch für einen Fügeprozess vorbehandelt bzw. vorbereitet. Diese Vorbehandlung führt dazu, dass der sich anschließende Fügeprozess verbessert bzw. stabiler gestaltet werden kann. Dieses bewirkt, dass sich die Durchlaufzeit für die Herstellung eines medizinischen Implantats verringert, da weniger Nachbearbeitungsschritte notwendig sind. Zudem wird für das Fügen eine höhere Prozesssicherheit erreicht.

Der Vorteil der Plasmaaktivierung im Niederdruckplasma besteht außerdem darin, dass dieses Plasma kriechgängig ist, d. h. dass dieses Plasma es ermöglicht, auch Hinterschneidungen zu aktivieren.

Besonders bevorzugt weist die Oberfläche des ersten Elements und des mindestens einen zweiten Elements Kunststoff, der vorzugsweise ausgewählt ist aus einem oder mehreren Polymeren der Gruppen Polyurethan, Polycarbonat, Polyester, Polyether, Polyolefin, insbesondere Polyethylen und Polypropylen, Polyvinylchlorid (PVC), Polystyrol, und/oder einen metallischen Werkstoff, der vorzugsweise ausgewählt ist aus einem oder mehreren metallischen Werkstoffen der Gruppe Titan, Aluminium, Edelstahl, vorzugsweise 316L und 316LVM, Kobalt-Nickel-Legierung, vorzugsweise MP35N, Silber, Gold, Platin, Iridium, Niob, L 605, und/oder einen keramischen Werkstoff, vorzugsweise Aluminiumoxid, auf. Diese Materialen haben sich im Zusammenhang mit der Herstellung von medizinischen Implantaten als besonders geeignet für den Einsatz im menschlichen Körper erwiesen.

In einem bevorzugten Ausführungsbeispiel der Erfindung beinhaltet der Fügeschritt einen Klebeschritt, wobei als Klebemittel vorzugsweise Epoxidharz oder Silikon verwendet wird. Das Kleben ist ein besonders einfacher Fügeschritt, der zu einer dauerhaft festen und bei Verwendung eines geeigneten Klebemittels zu einer gegenüber Gasen oder Körperflüssigkeiten dichten Verbindung führt.

In einem weiteren besonders bevorzugten Ausführungsbeispiel der Erfindung werden das erste Element und das mindestens eine zweite Element vor dem Fügeschritt und dem Plasmaaktivierungsschritt miteinander verschweißt und/oder montiert. Durch Verschweißen können beispielsweise elektrisch leitende Anschlüsse des ersten Elements und des mindestens einen zweiten Elements miteinander verbunden werden. Beim Montieren kann beispielsweise durch Verwendung von Zapfen oder Stiften an dem einen Element und entsprechenden Öffnungen an dem anderen Element eine kraft- und/oder formschlüssige Vorverbindung zwischen den Elementen erreicht werden, so dass die Elemente vor dem Fügen und dem Plasmaaktivierungsschritt bereits in der richtigen Lage zueinander angeordnet sind. Hierdurch wird der anschließende Plasmaaktivierungsschritt und vor allem der Fügeschritt erleichtert.

Für den Plasmaaktivierungsschritt haben sich als besonders geeignete Plasmen die Plasmen erwiesen, die mindestens ein Element oder eine Verbindung der Gruppe Sauerstoff, Wasserstoff, Argon, Stickstoff, Ammoniak und Fluor enthalten.

Vorzugsweise wird das Plasma des Plasmaaktivierungsschritts bei einer Frequenz von etwa 5 kHz bis etwa 100 kHz oder bei einer Frequenz von 13,56 MHz oder 2,45 GHz betrieben. Weiterhin bevorzugt weisen die für den Plasmaaktivierungsschritt verwendeten Plasmen eine Leistung von etwa 1 W bis etwa 1000 W auf. Die Behandlungsdauer des/der im Plasma behandelten Elements/Elemente beträgt vorzugsweise etwa 10 Sekunden bis etwa 30 Minuten. In dieser Zeit wird einerseits eine ausreichende Aktivierung der Oberfläche erreicht und andererseits ein zu großer Abtrag der Oberfläche verhindert.

Um die im Plasma durchgeführte Aktivierung der Oberfläche des Elements/der Elemente optimal zu nutzen, muss der Fügeschritt vorzugsweise frühestens etwa 15 Sekunden bis spätestens etwa 300 Minuten nach dem Plasmaaktivierungsschritt begonnen werden.

Besonders bevorzugt wird unmittelbar vor dem Plasmaaktivierungsschritt mindestens ein Teilbereich des Verbindungsbereichs des ersten Elements und/oder des mindestens einen zweiten Elements in einem Plasmareinigungsschritt mittels eines bei Atmosphärendruck erzeugten Plasmas (Normaldruckplasma) gereinigt. Dieser Verfahrensschritt kann auch als Plasmaätzen bezeichnet werden. Dieser zusätzliche Schritt ist insbesondere dann sinnvoll, wenn Teile der miteinander zu verbindenden Elemente, beispielsweise elektrisch leitende Anschlüsse des Gehäuses und des Headers eines Herzschrittmachers vor dem Plasmaaktivierungsschritt miteinander verschweißt wurden. Beim Schweißen entsteht Ruß, beispielsweise durch Verbrennung von Fett, das auf der Oberfläche abgelagert ist. Dieser Ruß kann zu Überschlägen zwischen den Leiterbahnen führen und hierdurch die mit den Anschlüssen verbundene Elektronik zerstören. Um diese Folgen zu vermeiden, kann dieser Ruß oder andere Oberflächenablagerungen mittels des Plasmareinigungsschritts, der lokal begrenzt durchgeführt werden kann, entfernt werden.

In einem bevorzugten Ausführungsbeispiel der Erfindung wird der Plasmareinigungsschritt in einem Plasma durchgeführt, das Sauerstoff und/oder Fluor enthält.

Der Abtrag von Material im Plasmareinigungsschritt wird vorzugsweise dadurch begrenzt, dass die Behandlungsdauer des/der im Plasma beim Plasmareinigungsschritt behandelten Elements/Elemente maximal etwa 15 Minuten beträgt.

Vorzugsweise wird das Plasma des Plasmareinigungsschritts bei einer Frequenz von 27,2 MHz oder bei einer Frequenz von 2,45 GHz betrieben.

Besonders bevorzugt ist das erste Element der Header und das zweite Element das Gehäuse und/oder das zweite Element der Deckel eines Herzschrittmachers. Hierbei ist der Header eine Bezeichnung für ein Element des Herzschrittmachers, der den Verbindungskopf bildet. Der Header dient zum Einstecken der Elektrode(n) in die entsprechende(n) Steckeraufnahme(n) und zur Verbindung der Elektrode(n) mit dem Gehäuse. Hierfür weist der Header (einen) entsprechende(n) Anschlüsse/Anschluss auf. Das Gehäuse enthält einerseits die Batterien des Herzschrittmachers und andererseits die elektronischen Einrichtungen zur Übertragung und Verarbeitung der mit der/den Elektrode(n) gemessenen Daten aus dem Herzen sowie zur Berechnung der von der/den Elektrode(n) zu sendenden Impulse zur Stimulierung des Herzens. Nach der Verbindung des Headers mit dem Gehäuse sind die elektronischen Einrichtungen des Gehäuses elektrisch mit den Enden der Elektrode(n) verbunden. Hierfür weist das Gehäuse einen Anschluss/Anschlüsse auf, die mit dem(n) entsprechenden Anschluss/Anschlüssen des Headers elektrisch verbunden werden. Der Deckel dient zur Abdeckung und Abdichtung der Steckeraufnahme(n) des Headers.

Im Hinblick auf die Herstellung des Herzschrittmachers aus den Elementen Deckel, Header und Gehäuse wird das folgende Verfahren erfindungsgemäß bevorzugt angewendet:
a) Zunächst werden der Header und der Deckel einzeln dem Plasmaaktivierungsschritt unterzogen,
b) anschließend werden der Header und der Deckel montiert und danach in einem ersten Fügeschritt miteinander verklebt,
c) nun wird die Einheit aus Header und Deckel auf das Gehäuse montiert, der/die Anschluss/Anschlüsse von Gehäuse und Header werden miteinander verschweißt und anschließend Gehäuse und Header in einem zweiten Fügeschritt miteinander verklebt.

Ebenfalls als vorteilhaft hat sich ein Verfahren erwiesen, bei dem
d) zunächst der Header und der Deckel miteinander montiert und als montierte Einheit dem Plasmaaktivierungsschritt unterzogen werden,
e) anschließend der Header und der Deckel in dem ersten Fügeschritt miteinander verklebt werden,
c) nun die Einheit aus Header und Deckel auf das Gehäuse montiert wird und der/die Anschluss/Anschlüsse von Gehäuse und Header miteinander verschweißt wird/werden und anschließend Gehäuse und Header in dem zweiten Fügeschritt miteinander verklebt werden.

Weiter bevorzugt wird ein Verfahren angewendet, bei dem
d) zunächst der Header und der Deckel miteinander montiert und als montierte Einheit dem Plasmaaktivierungsschritt unterzogen werden,
e) anschließend der Header und der Deckel in dem ersten Fügeschritt miteinander verklebt werden,
f) nun die Einheit aus Header und Deckel auf das Gehäuse montiert wird und der/die Anschluss/Anschlüsse von Gehäuse und Header miteinander verschweißt wird/werden und
g) anschließend die Einheit aus Gehäuse und Header mit Deckel einem weiteren Plasmaaktivierungsschritt unterzogen und
h) danach in dem zweiten Fügeschritt miteinander verklebt werden.

In dem zuletzt aufgeführten Verfahren hat es sich als vorteilhaft erwiesen, wenn das Gehäuse und der Header vor dem Schritt g) und nach dem Schritt f) im Bereich der verschweißten Anschlüsse einem zusätzlichen Plasmareinigungsschritt unterzogen wird.

Der Reinigungsvorgang vermindert Nacharbeiten bedingt durch Rußrückstände und verbessert die Sicherheit vor Spannungsüberschlägen zwischen den spannungsführenden Bauteilen der Aussenverdrahtung und der Durchführung.

Bei dem erfindungsgemäßen Verfahren ist es außerdem möglich, ein Element oder mehrere Elemente einem Plasmaaktivierungsschritt oder einem Plasmareinigungsschritt mehrfach zu unterziehen.

Die obige Aufgabe wird außerdem durch ein medizinisches Implantat gelöst, das nach einem der oben angegebenen Verfahren hergestellt ist. Ein derartiges medizinisches Implantat zeichnet sich dadurch aus, dass es kostengünstig hergestellt werden kann und seine Fehleranfälligkeit verringert ist.

Weitere Ziele, Merkmale, Vorteile und Anwendungsmöglichkeiten der Erfindung ergeben sich aus der nachfolgenden Beschreibung von Ausführungsbeispielen anhand der Figuren. Dabei bilden alle beschriebenen und/oder bildlich dargestellten Merkmale für sich oder in beliebiger Kombination den Gegenstand der vorliegenden Erfindung, unabhängig von ihrer Zusammenfassung in den einzelnen Ansprüchen oder deren Rückbeziehung.

Es zeigen schematisch:
- Figur 1: ein Gehäuse eines Herzschrittmachers in einer Ansicht von der Seite,
- Figur 2: einen Header eines Herzschrittmachers in einer Ansicht von der Seite,
- Figur 3: einen Deckel für zwei Steckeraufnahmen des Headers in einer Ansicht von oben,
- Figur 4: die Anordnung eines Headers gemäß Figur 2 und eines Deckels gemäß Figur 3 in einer Plasmaaktivierungs-Vorrichtung,
- Figur 5: den Header gemäß Figur 2 versehen mit dem Deckel gemäß Figur 3 in einer Ansicht von der Seite,
- Figur 6: eine Einheit aus Header und Deckel gemäß Figur 5 und dem Gehäuse gemäß Figur 1 in einer Ansicht von der Seite,
- Figur 7: die Anordnung einer Einheit gemäß Figur 6 in einer Plasmaaktivierungs-Vorrichtung und
- Figur 8: die Anordnung einer Einheit gemäß Figur 6 in einer Vorrichtung zur Durchführung des Reinigungsschrittes.

Im Folgenden soll das erfindungsgemäße Verfahren zur Herstellung eines medizinischen Implantats am Beispiel der Herstellung eines Herzschrittmachers mit den Elementen Gehäuse 10, Header 20 und Deckel 30 dargestellt werden.

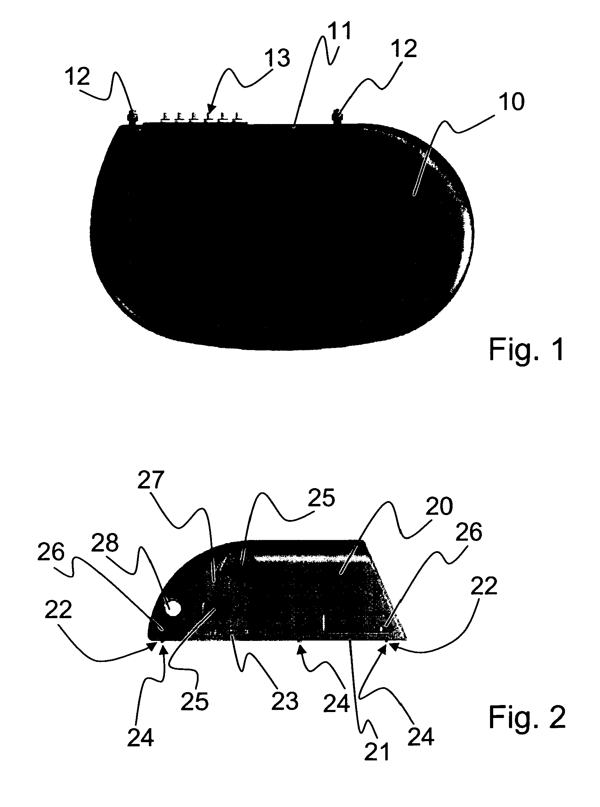

Das Gehäuse 10 ist in Figur 1 dargestellt und weist eine flache, in etwa ovale Form auf. An seiner Oberseite ist es mit einer geraden verlaufenden Kante 11 versehen. Diese Kante 11 ist zur Anordnung eines Headers 20 vorgesehen und weist zwei stiftförmige, zylindrische Zapfen 12 auf, die jeweils an gegenüber liegenden Enden der Kante 11 angeordnet sind. Diese stiftförmigen Zapfen 12 dienen zur Montage des Headers 20. Hierfür weisen sie in ihrem mittleren Abschnitt einen im Durchmesser vergrößerten Abschnitt auf, der von dem darüber und dem darunter liegenden Abschnitt jeweils durch eine Stufe abgegrenzt ist.

Außerdem sind an der Kante 11 sechs Anschlüsse 13 vorgesehen, die zu einer elektrisch leitenden Verbindung mit entsprechenden Anschlüssen des Headers 20 dienen. In weiteren erfindungsgemäßen Ausführungsbeispielen kann auch lediglich ein Anschluss oder zwei bis fünf oder mehr als sechs Anschlüsse 13 vorgesehen sein. Zur Isolierung gegenüber dem Gehäuse 10, das an seiner Oberfläche meist aus einem metallischen Material, z. B. einem Edelstahl oder Titan, besteht, weisen die Anschlüsse 13 je ein zylindrisches, vorzugsweise keramisches, Isolierelement auf, das den jeweiligen stiftförmigen, elektrisch leitenden Anschluss im Bereich des Austritts des Stiftes aus dem Gehäuse 10 umgibt.

Wie oben bereits dargestellt enthält das Gehäuse 10 die Batterien des Herzschrittmachers sowie elektronischen Einrichtungen.

Der in Figur 2 dargestellte Header 20 weist Aufnahmen 22 auf, in die die Zapfen 12 des Gehäuses 10 bei der Montage eingesteckt werden können.

Im Bereich der unteren, gerade verlaufenden Kante 21 des Headers 20 sind sechs elektrisch leitende Anschlüsse 23 vorgesehen, die stiftförmig ausgebildet sind und nach unten aus dem Header 20 herausragen. Diese werden bei Herstellung des Herzschrittmachers mit den entsprechenden Anschlüssen 13 des Gehäuses 10 verbunden.

An der Kante 21 sind außerdem über die gesamte Länge der Kante 21 verteilt drei kurze Stifte (Zapfen) 24 vorgesehen, die ebenfalls der Montage des Headers 20 an dem Gehäuse 10 dienen. Die Zapfen 12 und die Stifte 24 können auch als Ankerstifte bzw. Fixierhilfe bezeichnet werden.

Der Header 20 weist ferner auf einer die beiden Steckeraufnahmen 25 umgebenden ca. ovalen Linie einen Absatz 27 auf, der zur Befestigung des entsprechend geformten Deckels 30 zur Abdeckung der Steckeraufnahmen 25 dient. Vorzugsweise weist der Absatz 27 hierfür eine Hinterschneidung auf.

An dem Header 20 sind im Bereich seiner linken und seiner rechten unteren Ecke Öffnungen 26 zum Entweichen von Luft aus dem Header sowie eine durchgehende, kreisrunde Ausnehmung 28 vorgesehen, die zum Vernähen des Herzschrittmachers im Körper des Patienten dient. An dem Header 20 kann außerdem eine Markierung zur Erkennung des Herzschrittmachers mittels Röntgenstrahlung vorgesehen sein.

In weiteren Ausführungsbeispielen kann der Header 20 je nach Bedarf auch lediglich eine Steckeraufnahme 25 oder mehr als zwei Steckeraufnahmen 25 aufweisen. Die Form des Absatzes 27 und somit die Form des entsprechenden Deckels 30 ist entsprechend der Anzahl der Steckeraufnahmen 25 gestaltet. In weiteren Ausführungsbeispielen können beispielsweise jeweils zwei Steckeraufnahmen 25 zusammengefasst und von einem Absatz 27 umgeben und entsprechend von einem Deckel 30 abgedeckt werden.

Der Header 20 weist in dem in Figur 2 dargestellten Ausführungsbeispiel sechs Anschlüsse 23 auf. In weiteren Ausführungsbeispielen kann der Header 20 auch lediglich einen Anschluss 23 oder eine beliebige andere Anzahl von Anschlüsse 23 aufweisen.

Die Oberfläche des Headers 20 besteht vorzugsweise aus Polyurethan, Polycarbonat, Polyester, Polyether oder Polyolefin, insbesondere aus Polyethylen und Polypropylen, aus Polyvinylchlorid (PVC) oder Polystyrol.

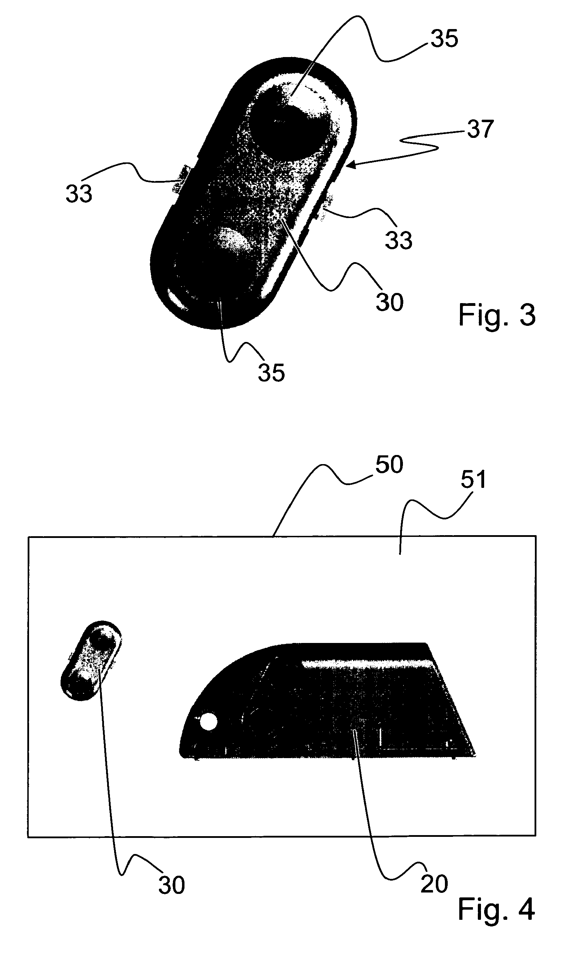

Der in Figur 3 dargestellte Deckel 30 dient zur Abdeckung der beiden Steckeraufnahmen 25 des Headers 20. Hierfür weist der Deckel 30 zwei kreisrunde Ausformungen 35 auf, in denen mittig ein Schlitz vorgesehen ist, durch den der nicht dargestellte Stecker der jeweiligen Elektrode hindurch in die jeweilige, darunter liegende Steckeraufnahme gesteckt werden kann. Hierdurch wird die Verbindung zwischen Elektrodenstecker und Steckeraufnahme 25 abgedichtet.

Seitlich, jeweils an den Längsseiten des Rands 37, weist der Deckel je eine Lasche 33 auf, welche zur Montage des Deckels 30 an dem Header 20 dient. Der den Deckel 30 umgebende Rand 37 ist derart gestaltet, dass er an dem Absatz 27 des Headers 20 befestigt werden kann. Der Rand 37 weist hierfür vorzugsweise eine Hinterschneidung auf.

Die Oberfläche des Deckels 30 besteht aus Polyurethan, Polycarbonat, Polyester oder Polyether, Polyolefin, insbesondere aus Polyethylen und Polypropylen, Polyvinylchlorid (PVC) oder Polystyrol.

An dem Header 20 können im Bereich des Absatzes 27 und/oder an dem Deckel 30 im Bereich des Randes 37 Dichtelemente, vorzugsweise Dichtlippen, vorgesehen sein, die zur zusätzlichen Abdichtung von Header 20 und Deckel 30 in diesem Bereich dienen.

Nachdem jedes der Elemente Gehäuse 10, Header 20 und Deckel 30 für sich gefertigt wurde, geht es nun darum, diese drei Elemente zu einem Herzschrittmacher zusammenzufügen.

Hierzu werden in einem ersten Ausführungsbeispiel des erfindungsgemäßen Herstellungsverfahrens *(**Aktivierungsvorgang 01**)* zunächst Header 20 und Deckel 30 separat in einer Vorrichtung 50 zur Plasmaaktivierung angeordnet, um die Oberfläche von Header 20 und Deckel 30 in einem Plasmaaktivierungsschritt zu aktivieren. Die in Figur 4 schematisch dargestellte Plasmaaktivierungs-Vorrichtung 50 nimmt hierfür den Header 20 und den Deckel 30 vollständig auf. Im Inneren der Plasmaaktivierungs-Vorrichtung ist zwischen zwei nicht dargestellten Elektroden (Kathode und Anode) ein Plasma 51 angeordnet, das die Elemente Header 20 und Deckel 30 umgibt.

Da die Erzeugung eines Plasmas an sich bekannt ist, soll hierauf im Folgenden nicht näher eingegangen werden.

In der Vorrichtung 50 wird ein Niederdruckplasma oder ein Normaldruckplasma (bei Atmosphärendruck erzeugtes Plasma) erzeugt, wobei in dem Plasma mindestens ein Element oder eine Verbindung der Gruppe Sauerstoff, Wasserstoff, Argon, Stickstoff, Ammoniak und Fluor enthalten ist. Das Plasma wird vorzugsweise bei einer Frequenz von etwa 5 kHz bis etwa 100 kHz oder einer Frequenz von 13,56 MHz oder 2,45 GHz betrieben. Hierbei gibt das Plasma vorzugsweise eine Leistung von 1 W bis etwa 1000 W ab. Die Behandlungsdauer des Headers 20 und des Deckels 30 im Plasma beträgt etwa 10 Sekunden bis etwa 30 Minuten.

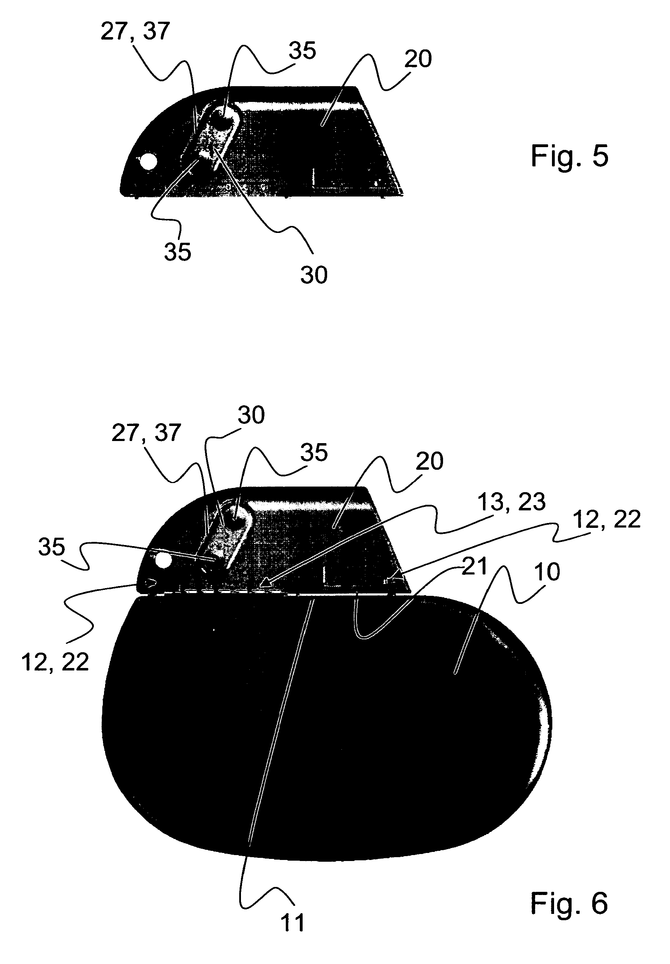

Nachdem Header 20 und Deckel 30 in dem Plasmaaktivierungsschritt behandelt wurden, wird der Deckel 30 auf den Header 20 montiert, d. h. die Laschen 33 des Deckels 30 werden in entsprechenden Öffnungen des Headers 20 angeordnet. Anschließend erfolgt das Verkleben des Deckels 30 mit dem Header 20, wobei hierfür ein Klebemittel entlang des Absatzes 27 bzw. des Randes 37 platziert wird. Das Klebemittel, vorzugsweise Epoxidharz oder Silikon, erzeugt einerseits eine feste Verbindung zwischen Deckel 30 und Header 20 und dichtet andererseits den Bereich zwischen Header 20 und Deckel 30 ab. Bei dem Klebeschritt ist darauf zu achten, dass dieser Fügeschritt frühestens etwa 15 Sekunden bis spätestens etwa 300 Minuten nach dem Plasmaaktivierungsschritt begonnen wird. Die resultierende Einheit aus Header 20, der mit dem Deckel 30 verklebt ist, zeigt Figur 5.

Anschließend wird der mit dem Deckel 30 versehene Header 20 mit seiner unteren Kante 21 auf die obere Kante 11 des Gehäuses 10 gesetzt und so montiert, dass die Stifte 24 des Headers 20 in entsprechenden Aufnahmen des Gehäuses 10 und jeder Zapfen 12 des Gehäuses 10 in der entsprechenden Aufnahme 22 des Headers 20 angeordnet sind. Außerdem wird jeder Anschluss 13 des Gehäuses 10 mit jeweils einem entsprechenden, gegenüber liegenden Anschluss 23 des Headers 20 verschweißt. Der Zustand nach der Montage des mit dem Deckel 30 verklebten Headers 20 auf das Gehäuse 10 und der Verschweißung der Anschlüsse 13, 23 ist in Figur 6 dargestellt. Der Deckel 30, der Header 20 und das Gehäuse 10 bilden hierbei eine Einheit, die nicht mehr ohne Weiteres getrennt werden kann.

Nach dem Verschweißen und der Montage von Header 20 und Gehäuse 10 werden diese Elemente miteinander verklebt. Hierfür wird ein Klebemittel, vorzugsweise Epoxidharz oder Silikon, in dem Bereich des Zwischenraums zwischen den Kanten 11, 21 derart angeordnet, dass das Klebemittel den Zwischenraum verschließt. Außerdem werden die verbundenen Anschlüsse 13, 23 abgedeckt und durch das Klebemittel die nebeneinander liegenden verbundenen Anschlüsse 13, 23 zueinander isoliert. Das Klebemittel wird zudem derart eingebracht, dass es in den Header 20 eindringt und nach außen durch die Öffnungen 26 austritt. Hierdurch wird sichergestellt, dass sich das Klebemittel wie gewünscht vollständig im Bereich der Kanten 11, 21 und der Anschlüsse 13, 23 bzw. im unteren Bereich des Headers 20 ausgebreitet hat und die gewünschte Abdichtung in dem Bereich erreicht wurde. Die Herstellung des Herzschrittmachers nach dem ersten erfindungsgemäßen Ausführungsbeispiel ist hiermit beendet.

In einem zweiten bevorzugten Ausführungsbeispiel (***Aktivierungsvorgang 03***) können Header 20 und Deckel 30 auch derart miteinander verbunden werden, dass sie zunächst aufeinander montiert werden und anschließend in diesem montierten Zustand gemeinsam plasmaaktiviert werden. Anschließend werden diese verklebt. Das Verfahren zur Verbindung des Headers 20 mit dem Gehäuse 10 verläuft analog zum ersten Ausführungsbeispiel.

In einem dritten bevorzugten Ausführungsbeispiel des erfindungsgemäßen Verfahrens *(Aktivierungsvorgang 02)* kann der Header 20 nach separater Plasmaaktivierung von Header 20 und Deckel 30 auch zunächst auf das Gehäuse 10 wie oben beschrieben montiert werden. Anschließend werden wie oben beschrieben die Anschlüsse 13, 23 von Gehäuse 10 und Header 20 miteinander verschweißt und Header 20 und Gehäuse 10 wie oben beschrieben miteinander verklebt. In einem weiteren Verfahrensschritt wird der plasmaaktivierte Deckel 30 wie oben beschrieben auf den Header 20 montiert und nochmals zusammen mit dem bereits mit dem Header 20 verklebten Gehäuse 10 in der Plasmaaktivierungs-Vorrichtung 50 plasmaaktiviert. Nach erfolgter Plasmaaktivierung werden Deckel 30 und Header 20 wie oben beschrieben verklebt.

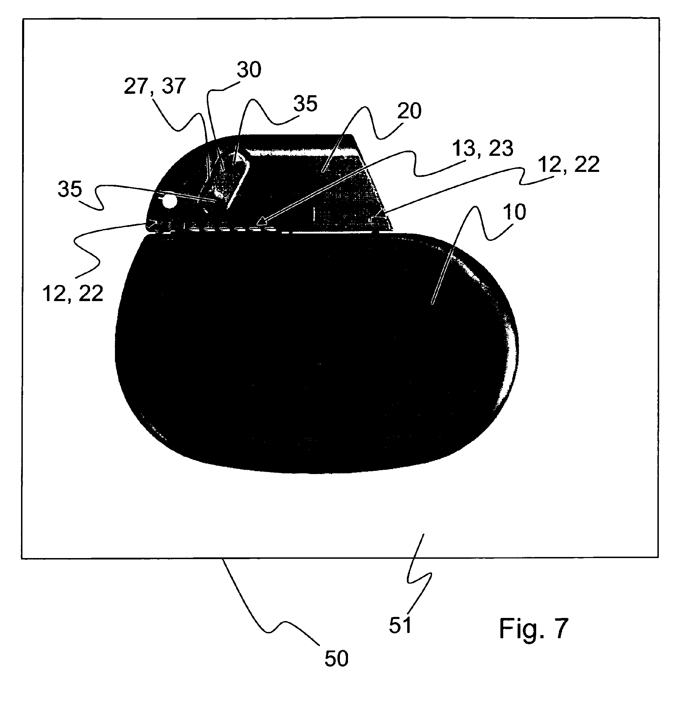

In einem vierten Ausführungsbeispiel **(*Aktivierungsvorgang 04*)** eines erfindungsgemäßen Verfahrens werden zunächst Header 20 und Deckel 30 wie oben beschrieben montiert und im montierten Zustand gemeinsam als Einheit plasmaaktiviert. Anschließend werden Header 20 und Deckel 30 miteinander wie oben beschrieben verklebt. In einem sich daran anschließenden Schritt wird der mit dem Deckel 30 versehene Header 20 auf das Gehäuse 10 wie oben beschrieben montiert und die Anschlüsse 13, 23 von Gehäuse 10 und Header 20 verschweißt. In diesem Zustand wird diese Einheit, die die miteinander verklebten Elemente Header 20 und Deckel 30, das mit dem Header 20 montierte Gehäuse 10 und verschweißte Anschlüsse 13, 23 aufweist, einem weiteren Plasmaaktivierungsschritt unterzogen, der in Figur 7 dargestellt ist. Diese Figur zeigt die Anordnung der genannten Einheit in der Vorrichtung 50 zur Plasmaaktivierung.

Die in Figur 7 gezeigte Vorrichtung zur Plasmaaktivierung 50 entspricht der in Figur 4 dargestellten Vorrichtung 50 in Bezug auf ihre im Zusammenhang mit Figur 4 genannten Eigenschaften. Da die Einheit aus den Elementen Gehäuse 10, Header 20 und Deckel 30 eine größere Ausdehnung als Header 20 und Deckel 30 separat aufweist, ist es lediglich notwendig, den Innenraum der Vorrichtung zur Plasmaaktivierung in einer entsprechenden Größe vorzusehen.

Nach der in Figur 7 dargestellten Plasmaaktivierung werden Header 20 und Gehäuse 10 wie oben beschrieben miteinander verklebt.

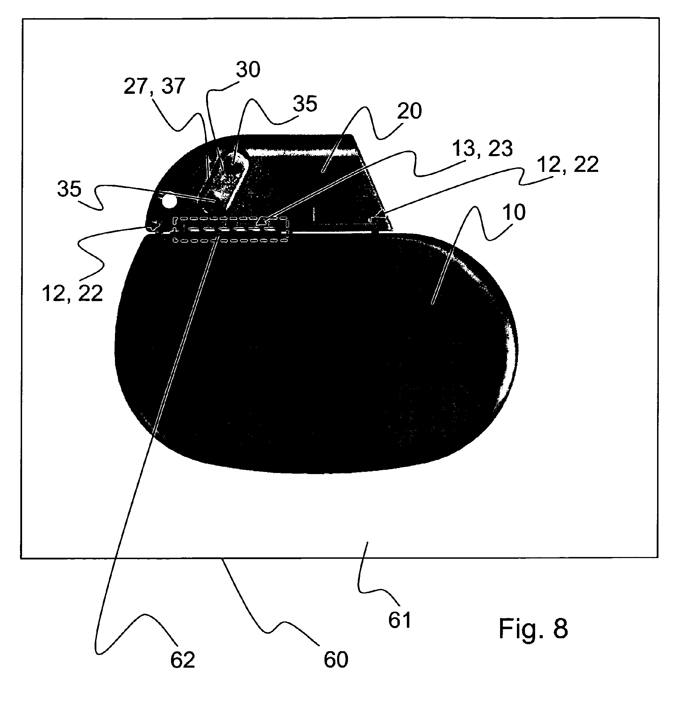

In einem fünften bevorzugten Ausführungsbeispiel (***Aktivierungsvorgang 05***), das analog zum vierten Ausführungsbeispiel durchgeführt wird, kann zusätzlich vor einer Plasmaaktivierung von aufeinander montiertem Header 20 und Gehäuse 10, die auch bereits verschweißte Anschlüsse 13, 23 aufweisen, zusätzlich ein Plasmareinigungsschritt vorgesehen werden, der schematisch in Figur 8 dargestellt ist.

Figur 8 zeigt die analog zur Einheit aus Figur 7 aufgebaute Einheit mit Header 20 mit verklebtem Deckel 30 und an den Header montiertem Gehäuse 10, wobei die Anschlüsse 13, 23 bereits miteinander verschweißt wurden, in der Vorrichtung 60 zur Plasmareinigung. Die Vorrichtung 60 weist zur Plasmareinigung ein Plasma 61 auf. In dem Bereich 62 der miteinander verschweißten Anschlüsse 13, 23 wird die Einheit aus Header 20, Gehäuse 10 und Deckel 30 in einem bei Atmosphärendruck erzeugten Plasma (Normaldruckplasma) gereinigt. Dies bedeutet, dass beispielsweise bei dem Verschweißen der Anschlüsse 13, 23 entstandener Ruß durch die Plasmareinigung abgetragen und von der Einheit weg transportiert wird. Das in der Vorrichtung 60 enthaltende Plasma enthält vorzugsweise Sauerstoff und/oder Fluor. Die Plasmareinigungs-Behandlung im Bereich 62 dauert maximal etwa 12 Minuten. Das in der Vorrichtung 60 angeordnete Plasma 61 wird bei einer Frequenz von 27,2 MHz oder bei einer Frequenz von 2,45 GHz betrieben.

Wie im vierten Ausführungsbeispiel beschrieben wird das Herstellungsverfahren nach der Reinigung im Plasmareinigungsschritt mit der Plasmaaktivierung und dem Kleben von Header 20 und Gehäuse 10 fortgesetzt.

In einem sechsten Ausführungsbeispiel (***Aktivierungsvorgang 06***) kann der nach der Plasmareinigung durchgeführte weitere Plasmaaktivierungsschritt im fünften Ausführungsbeispiel auch weggelassen werden. Ferner kann das Montieren und Verkleben von Header 20 und Deckel 30 erst nach dem Verkleben von Header 20 und Gehäuse 10 erfolgen.

In einem siebten Ausführungsbeispiel (***Aktivierungsvorgang 07 ohne Plasmareinigung***) können auch der Deckel 30 auf dem Header 20 montiert werden und der mit dem montierten Deckel versehene Header 20 auf das Gehäuse 10 montiert werden. Anschließend werden die Anschlüsse 13, 23 von Header 20 und Gehäuse 10 miteinander verschweißt. Die so montierte und verschweißte Einheit aus Deckel 30, Header 20 und Gehäuse 10 wird anschließend gemeinsam einem Plasmaaktivierungsschritt unterzogen. Anschließend werden, wie oben beschrieben, Gehäuse 10 und Header 20 miteinander und Deckel 30 und Header 20 miteinander verklebt.

In einem achten Ausführungsbeispiel **(*Aktivierungsvorgang 07*),** das sonst analog zum siebten Ausführungsbeispiel durchgeführt wird, kann der Bereich 62 der verschweißten Anschlüsse 13, 23 nach dem Verschweißen und vor der Plasmaaktivierung einem Plasmareinigungsschritt, wie oben im Zusammenhang mit dem fünften Ausführungsbeispiel beschrieben, unterzogen werden.

### Bezugszeichenliste:

- 10: Gehäuse
- 11: Kante
- 12: Zapfen
- 13: Anschluss
- 20: Header
- 21: Kante
- 22: Aufnahme
- 23: Anschluss
- 24: Stift
- 25: Steckeraufnahme
- 26: Öffnung
- 27: Absatz
- 28: Ausnehmung
- 30: Deckel
- 33: Lasche
- 35: Ausformung
- 37: Rand
- 50: Vorrichtung zur Plasmaaktivierung
- 51: Plasma
- 60: Vorrichtung zur Plasmareinigung
- 61: Plasma

## Patentansprüche

1. Verfahren zur Herstellung eines medizinischen Implantats, vorzugsweise eines Herzschrittmachers oder eines Defibrillators, aus einem ersten Element (10, 20, 30) und mindestens einem zweiten Element (10, 20, 30), die jeweils eine Oberfläche aufweisen, wobei das erste Element (10, 20, 30) und das mindestens eine zweite Element (10, 20, 30) in einem Fügeschritt in dem jeweiligen, mindestens einen Teilbereich der jeweiligen Oberfläche bildenden Verbindungsbereich des ersten Elements (10, 20, 30) und des mindestens einen zweiten Elements (10, 20, 30) miteinander verbunden werden, **dadurch gekennzeichnet, dass** in einem vor dem Fügeschritt durchzuführenden Plasmaaktivierungsschritt mindestens der Verbindungsbereich des ersten Elements (10, 20, 30) und/oder mindestens der Verbindungsbereich des mindestens einen zweiten Elements (10, 20, 30) in einem Niederdruckplasma oder in einem bei Atmosphärendruck erzeugten Plasma (51) aktiviert wird.

2. Verfahren nach Anspruch 1, **dadurch gekennzeichnet, dass** die Oberfläche des ersten Elements (10, 20, 30) und die Oberfläche des mindestens einen zweiten Elements (10, 20, 30) Kunststoff, der vorzugsweise ausgewählt ist aus einem oder mehreren Polymeren der Gruppe Polyurethan, Polycarbonat, Polyester, Polyether, Polyolefin, Epoxidharz, insbesondere Polyethylen und Polypropylen, Polyvinylchlorid, Polystyrol, und/oder einen metallischen Werkstoff, der vorzugsweise ausgewählt ist aus einem oder mehreren metallischen Werkstoffen der Gruppe Titan, Aluminium, Edelstahl, vorzugsweise 316L und 316LVM, L 605 Kobalt-Nickel-Legierung, vorzugsweise MP35N, Silber, Gold, Platin, Iridium, Niob, und/oder einen keramischen Werkstoff, vorzugsweise Aluminiumoxid, aufweist.

3. Verfahren nach einem der vorhergehenden Ansprüche, **dadurch gekennzeichnet, dass** der Fügeschritt einen Klebeschritt beinhaltet, wobei als Klebemittel vorzugsweise Epoxidharz oder Silikon verwendet wird.

4. Verfahren nach einem der vorhergehenden Ansprüche, **dadurch gekennzeichnet, dass** das erste Element (10, 20, 30) und das mindestens eine zweite Element (10, 20, 30) vor dem Fügeschritt und dem Plasmaaktivierungsschritt miteinander verschweißt und/oder montiert werden.

5. Verfahren nach einem der vorhergehenden Ansprüche, **dadurch gekennzeichnet, dass** der Plasmaaktivierungsschritt in einem Plasma (51) durchgeführt wird, das mindestens ein Element oder eine Verbindung der Gruppe Sauerstoff, Wasserstoff, Argon, Stickstoff, Ammoniak und Fluor enthält.

6. Verfahren nach einem der vorhergehenden Ansprüche, **dadurch gekennzeichnet, dass** das Plasma (51) des Plasmaaktivierungsschritts bei einer Frequenz von etwa 5 kHz bis etwa 100 kHz oder bei einer Frequenz von 13,56 MHz oder 2,45 GHz betrieben wird.

7. Verfahren nach einem der vorhergehenden Ansprüche, **dadurch gekennzeichnet, dass** das Plasma (51) des Plasmaaktivierungsschritts mit einer Leistung von etwa 1 W bis etwa 1000 W betrieben wird.

8. Verfahren nach einem der vorhergehenden Ansprüche, **dadurch gekennzeichnet, dass** im Plasmaaktivierungsschritt die Behandlungsdauer des/der im Plasma (51) behandelten Elements/Elemente (10, 20, 30) etwa 10 Sekunden bis etwa 30 Minuten beträgt.

9. Verfahren nach einem der vorhergehenden Ansprüche, **dadurch gekennzeichnet, dass** der Fügeschritt frühestens etwa 15 Sekunden bis spätestens etwa 300 Minuten nach dem Plasmaaktivierungsschritt begonnen wird.

10. Verfahren nach einem der vorhergehenden Ansprüche, **dadurch gekennzeichnet, dass** unmittelbar vor dem Plasmaaktivierungsschritt mindestens ein Teilbereich (62) des Verbindungsbereichs des ersten Elements (10, 20, 30) und/oder des mindestens einen zweiten Elements (10, 20, 30) in einem Plasmareinigungsschritt mittels eines bei Atmosphärendruck erzeugten Plasmas (61) gereinigt wird.

11. Verfahren nach Anspruch 10, **dadurch gekennzeichnet, dass** der Plasmareinigungsschritt in einem Plasma (61) durchgeführt wird, das Sauerstoff und/oder Fluor enthält.

12. Verfahren nach Anspruch 11, **dadurch gekennzeichnet, dass** die Behandlungsdauer beim Plasmareinigungsschritt des/der im Plasma (61) behandelten Elements/Elemente (10, 20, 30) maximal etwa 15 Minuten beträgt.

13. Verfahren nach einem der Ansprüche 10 bis 11, **dadurch gekennzeichnet, dass** das Plasma (61) des Plasmareinigungsschritts bei einer Frequenz von 27,2 MHz oder bei einer Frequenz von 2,45 GHz betrieben wird.

14. Verfahren nach einem der vorhergehenden Ansprüche, **dadurch gekennzeichnet, dass** das erste Element der Header (20) und das zweite Element das Gehäuse (10) und/oder das zweite Element der Deckel (30) eines Herzschrittmachers oder Defibrillators ist.

15. Verfahren nach Anspruch 14, **dadurch gekennzeichnet, dass**
a) zunächst der Header (20) und der Deckel (30) einzeln dem Plasmaaktivierungsschritt unterzogen werden,
b) anschließend der Header (20) und der Deckel (30) montiert und danach in einem ersten Fügeschritt miteinander verklebt werden,
c) nun die Einheit aus Header (20) und Deckel (30) auf das Gehäuse (10) montiert wird und der/die Anschluss/Anschlüsse (13, 23) von Gehäuse (10) und Header (20) miteinander verschweißt wird/werden und anschließend Gehäuse (10) und Header (20) in einem zweiten Fügeschritt miteinander verklebt werden.

16. Verfahren nach Anspruch 14, **dadurch gekennzeichnet, dass**
d) zunächst der Header (20) und der Deckel (30) miteinander montiert und als montierte Einheit dem Plasmaaktivierungsschritt unterzogen werden,
e) anschließend der Header (20) und der Deckel (30) in dem ersten Fügeschritt miteinander verklebt werden,
c) nun die Einheit aus Header (20) und Deckel (30) auf das Gehäuse (10) montiert wird und der/die Anschluss/Anschlüsse (13, 23) von Gehäuse (10) und Header (20) miteinander verschweißt wird/werden und anschließend Gehäuse (10) und Header (20) in dem zweiten Fügeschritt miteinander verklebt werden.

17. Verfahren nach Anspruch 14, **dadurch gekennzeichnet, dass**
d) zunächst der Header (20) und der Deckel (30) miteinander montiert und als montierte Einheit dem Plasmaaktivierungsschritt unterzogen werden,
e) anschließend der Header (20) und der Deckel (30) in dem ersten Fügeschritt miteinander verklebt werden,
f) nun die Einheit aus Header (20) und Deckel (30) auf das Gehäuse (10) montiert wird und der/die Anschluss/Anschlüsse (13, 23) von Gehäuse (10) und Header (20) miteinander verschweißt wird/werden und
g) anschließend die Einheit aus Gehäuse (10) und Header (20) mit Deckel (30) einem weiteren Plasmaaktivierungsschritt unterzogen und
h) danach in dem zweiten Fügeschritt miteinander verklebt werden.

18. Verfahren nach einem der Ansprüche 15-17, **dadurch gekennzeichnet, dass** das Gehäuse (10) und der Header (20) im Bereich (62) der verschweißten Anschlüsse (13, 23) vor dem Plasmaaktivierungsschritt einem zusätzlichen Plasmareinigungsschritt unterzogen werden.

19. Medizinisches Implantat, vorzugsweise Herzschrittmacher oder Defibrillator, hergestellt nach einem der in den vorhergehenden Ansprüchen angegebenen Verfahren.
